(19) Europäisches Patentamt
European Patent Office
Office européen des brevets

(11) Veröffentlichungsnummer : **0 072 546**
**B1**

(12) # EUROPÄISCHE PATENTSCHRIFT

(45) Veröffentlichungstag der Patentschrift : **09.10.85**

(21) Anmeldenummer : **82107367.3**

(22) Anmeldetag : **13.08.82**

(51) Int. Cl.⁴ : **C 07 J 5/00**, A 61 K 31/57

(54) **Neue Hydrocortison-Derivate, ihre Herstellung und Verwendung.**

(30) Priorität : **18.08.81 DE 3133082**

(43) Veröffentlichungstag der Anmeldung : **23.02.83 Patentblatt 83/08**

(45) Bekanntmachung des Hinweises auf die Patenterteilung : **09.10.85 Patentblatt 85/41**

(84) Benannte Vertragsstaaten : **AT BE CH DE FR GB IT LI LU NL SE**

(56) Entgegenhaltungen :
**FR-A- 2 335 231**
**CHEMICAL ABSTRACTS, Band 86, Nr. 3, 17. Januar 1977, Seite 443, Nr. 16843p, Columbus, Ohio, USA**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Annen, Klaus, Dr.**
**Seegefelder Strasse 194**
**D-1000 Berlin 20 (DE)**
Erfinder : **Laurent, Henry, Dr.**
**Glambecker Weg 21**
**D-1000 Berlin 28 (DE)**
Erfinder : **Hofmeister, Helmut, Dr.**
**Weislingenstrasse 4**
**D-1000 Berlin 28 (DE)**
Erfinder : **Wiechert, Rudolf, Prof. Dr.**
**Petzower Strasse 8a**
**D-1000 Berlin 39 (DE)**
Erfinder : **Wendt, Hans, Dr.**
**Ernst-Ring Strasse 14**
**D-1000 Berlin 38 (DE)**

**EP 0 072 546 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue Hydrocortison-Derivate der allgemeinen Formel I

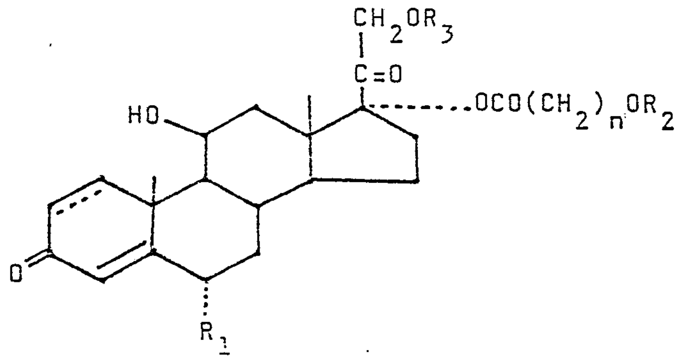

(I)

worin
..... eine Einfachbindung oder eine Doppelbindung,
n die Ziffern 1 oder 2,
$R_1$ ein Wasserstoffatom oder eine Methylgruppe,
$R_2$ einen Alkylrest mit 1 bis 6 Kohlenstoffatomen und
$R_3$ ein Wasserstoffatom oder einen 1-Oxoalkylrest mit 2 bis 6 Kohlenstoffatomen darstellen, ein Verfahren zu ihrer Herstellung und pharmazeutische Präparate, die diese Verbindungen als Wirkstoff enthalten.

Die neuen Hydrocortison-Derivate können als 2 bis 6 Kohlenstoffatome enthaltende 1-Oxoalkylgruppen $R_3$ beispielsweise eine Acetylgruppe, eine Propionylgruppe, eine Butyrylgruppe, eine Isobutyrylgruppe, eine Valerylgruppe, eine 3-Methylbutyrylgruppe, eine Trimethylacetylgruppe oder eine Hexanoylgruppe tragen.

Als Alkylgruppe $R_2$ können die Hydrocortison-Derivate beispielsweise eine Methylgruppe, eine Ethylgruppe, eine Propylgruppe, eine Isopropylgruppe, eine Butylgruppe, eine sec.-Butylgruppe, eine tert.-Butylgruppe oder eine Pentylgruppe tragen.

17α-Alkyloxyacetoxy-steroide und 17α-Acetyloxyacetoxy-steroide sind bereits vorbekannt. So sind beispielsweise in der französischen Patentschrift 2 335 231 gestagen und östrogen wirksame 17α-Acyloxyacetoxy-steroide beschrieben. In Chem. Abstr. *86*, 1977, 16843 werden antiinflammatorisch wirksame 17α-Alkyloxyacetoxy-Ester des Dexamethason beschrieben.

Die neuen Derivate des Hydrocortisons besitzen bei topischer Applikation eine starke antiinflammatorische Wirksamkeit und zeichnen sich gegenüber den vorbekannten strukturanalogen Kortikoiden durch eine günstigere Dissoziation zwischen erwünschter topischer und unerwünschter systemischer Wirksamkeit aus, was mit Hilfe des bekannten Vasokonstriktionstests und Adjuvans-Ödem-Tests ermittelt werden kann.

Die neuen Hydrocortison-Derivate der allgemeinen Formel I eigenen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel Lösungen, Lotionen, Salben, Cremes oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden, auch zur Behandlung allergischer Erkrankungen des Darmtraktes, wie der Kolitis ulcerosa und der Kolitis granulomatosa.

Die neuen Hydrocortison-Derivate können nach einem Verfahren hergestellt werden, welches unter den Bedingungen durchgeführt werden kann, wie sie in DE-A-2 645 104, -2 645 105 und -2 340 591 beschrieben sind, und das dadurch gekennzeichnet ist, daß man ein Hydrocortison-21-acylat der allgemeinen Formel II

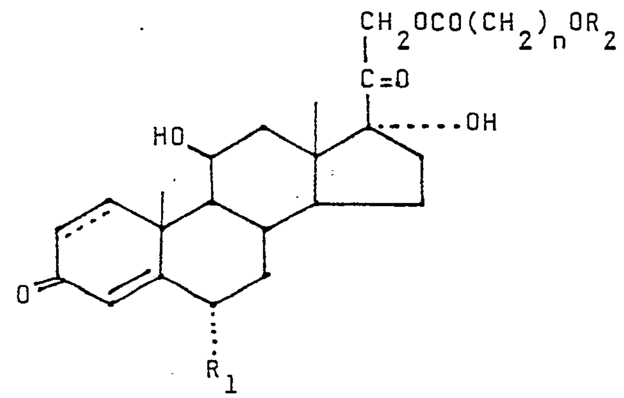

(II)

worin

....., $R_1$ und $R_2$ die obengenannte Bedeutung besitzen, zu dem entsprechenden 17-Acylat umlagert, gegebenenfalls in der 21-Position verestert und gewünschtenfalls die $\Delta^{1,4}$-Steroide der allgemeinen Formel I zu den $\Delta^4$-Steroiden hydriert.

Die nachfolgenden Ausführungsbeispiele dienen zur Erläuterung des erfindungsgemäßen Verfahrens.

Beispiel 1

a) 15,0 g 11β,17,21-Trihydroxy-4-pregnen-3,20-dion in 150 ml Pyridin werden bei 0 °C mit 9 ml Methoxyessigsäurechlorid 3 Stunden gerührt. Anschließend gibt man auf eine Eiswasser-Natriumchlorid-Lösung, filtriert den Niederschlag ab und reinigt an 1,1 kg Kieselgel mit einem Methylenchlorid-Aceton-Gradienten (0-20 % Aceton). Ausbeute 15,2 g 11β,17-Dihydroxy-21-methoxyacetoxy-4-pregnen-3,20-dion. Schmelzpunkt 232 °C.

b) Zu einer Suspension von 3,2 g Kupfer (I)-jodid in 64 ml wasserfreiem Tetrahydrofuran werden bei 0 °C unter Argon 20 ml einer 5 proz. Lösung von Methyllithium in Ether hinzugetropft. Die gelbe Lösung wird auf − 30 °C abgekühlt und eine Lösung von 2,9 g 11β,17-Dihydroxy-21-methoxyacetoxy-4-pregnen-3,20-dion in 100 ml wasserfreiem Tetrahydrofuran zugesetzt. Man rührt 20 Minuten bei − 30 °C weiter und gibt auf eine wäßrige Ammoniumchloridlösung. Nach der Extraktion mit Methylenchlorid wird die organische Lösung gewaschen, über Natriumsulfat getrocknet und im Vakuum eingedampft. Das Rohprodukt wird an 220 g Kieselgel mit einem Methylenchlorid-Aceton-Gradienten (0-25 % Aceton) gereinigt. Man isoliert 1,4 g 11β,21-Dihydroxy-17-methoxyacetoxy-4-pregnen-3,20-dion. Schmelzpunkt 185 °C.

Beispiel 2

a) Analog Beispiel 1a werden 10,0 g 11β,17,21-Trihydroxy-4-pregnen-3,20-dion in 100 ml Pyridin mit 6 ml Äthoxyessigsäurechlorid umgesetzt, aufgearbeitet und gereinigt. Ausbeute 10,3 g 21-Äthoxyacetoxy-11β,17-dihydroxy-4-pregnen-3,20-dion. Schmelzpunkt 203 °C.

b) Unter den Bedingungen des Beispiels 1b werden 10,0 g 21-Äthoxyacetoxy-11β,17-dihydroxy-4-pregnen-3,20-dion mit Lithiumdimethylcuprat umgelagert und aufgearbeitet. Das Rohprodukt wird an 700 g Kieselgel mit einem Hexan-Essigester-Gradienten (0-100 % Essigester) gereinigt. Man erhält 4,1 g 17-Äthoxyacetoxy-11β,21-dihydroxy-4-pregnen-3,20-dion. Schmelzpunkt 161 °C.

Beispiel 3

a) Wie im Beispiel 1a beschrieben, werden 20,0 g 11β,17,21-Trihydroxy-4-pregnen-3,20-dion in 200 ml Pyridin mit 3-Methoxypropionsäurechlorid umgesetzt, aufgearbeitet und gereinigt. Ausbeute 12,8 g 11β,17-Dihydroxy-21-(3-methoxypropionyloxy)-4-pregnen-3,20. Schmelzpunkt 188 °C.

b) Analog Beispiel 1b werden 11,5 g 11β,17-Dihydroxy-21-(3-methoxypropionyloxy)-4-pregnen-3,20-dion mit Lithiumdimethylcuprat umgelagert und aufgearbeitet. Nach der Reinigung an 750 g Kieselgel mit einem Methylenchlorid-Aceton-Gradienten (0-30 % Aceton) erhält man 7,3 g 11β,21-Dihydroxy-17-(3-methoxypropionyloxy)-4-pregnen-3,20-dion. Schmelzpunkt 172 °C.

Beispiel 4

a) Man setzt unter den Bedingungen des Beispiels 1a 10,0 g 11β,17,21-Trihydroxy-4-pregnen-3,20-dion mit 3-Äthoxypropionsäurechlorid um und chromatographiert an 1,5 kg Kieselgel mit einem Hexan-Essigester-Gradienten (0-70 % Essigester). Ausbeute 9,7 g 21-(3-Äthoxypropionyloxy)-11β,17-dihydroxy-4-pregnen-3,20-dion. Schmelzpunkt 126 °C.

b) 9,0 g 21-(3-Äthoxypropionyloxy)-11β,17-dihydroxy-4-pregnen-3,20-dion werden, wie in Beispiel 1b beschrieben, mit Lithiumdimethylcuprat umgelagert, aufgearbeitet und chromatographiert. Man isoliert 3,1 g 17-(3-Äthoxypropionyloxy)-11β,21-dihydroxy-4-pregnen-3,20-dion. Schmelzpunkt 149 °C.

Beispiel 5

a) Eine Lösung von 10,0 g 11β,17,21-Trihydroxy-1,4-pregnadien-3,20-dion in Pyridin wird analog Beispiel 1a mit Methoxyessigsäurechlorid umgesetzt, aufgearbeitet und gereinigt. Man isoliert 10,3 g 11β,17-Dihydroxy-21-methoxyacetoxy-1,4-pregnadien-3,20-dion.

b) 9,0 g 11β,17-Dihydroxy-21-methoxyacetoxy-1,4-pregnadien-3,20-dion werden analog Beispiel 1b mit Lithiumdimethylcuprat umgelagert, aufgearbeitet und gereinigt. Man erhält 3,6 g 11β,21-Dihydroxy-17-methoxyacetoxy-1,4-pregnadien-3,20-dion.

c) 1,5 g 11β,21-Dihydroxy-17-methoxyacetoxy-1,4-pregnadien-3,20-dion werden in 15 ml Pyridin und 7,5 ml Essigsäureanhydrid 2 Stunden bei Raumtemperatur gerührt. Anschließend gibt man auf eine Eiswasser-Natriumchlorid-Lösung, filtriert den Niederschlag ab und reinigt das Rohprodukt an 100 g Kieselgel mit einem Methylenchlorid-Aceton-Gradienten (0-20 % Aceton). Ausbeute 1,2 g 21-Acetoxy-11β-hydroxy-17-methoxyacetoxy-1,4-pregnadien-3,20-dion.

Beispiel 6

a) 10,0 g 11β,17,21-Trihydroxy-1,4-pregnadien-3,20-dion werden analog Beispiel 2a mit Äthoxyessigsäurechlorid umgesetzt, aufgearbeitet und gereinigt. Man isoliert 10,4 g 21-Äthoxyacetoxy-11β,17-dihydroxy-1,4-pregnadien-3,20-dion.

b) Analog Beispiel 2b werden 6,0 g 21-Äthoxyacetoxy-11β,17-dihydroxy-1,4-pregnadien-3,20-dion mit Lithiumdimethylcuprat umgelagert, aufgearbeitet und gereinigt. Ausbeute 2,5 g 17-Äthoxyacetoxy-11β,21-dihydroxy-1,4-pregnadien-3,20-dion.

Beispiel 7

a) Unter den Bedingungen des Beispiels 3a werden 10,0 g 11β,17,21-Trihydroxy-1,4-pregnadien-3,20-dion mit 3-Methoxypropionsäurechlorid umgesetzt, aufgearbeitet und gereinigt. Man isoliert 7,3 g 11β,17-Dihydroxy-21-(3-methoxypropionyloxy)-1,4-pregnadien-3,20-dion.

b) Analog Beispiel 1b werden 7,0 g 11β,17-Dihydroxy-21-(3-methoxypropionyloxy)-1,4-pregnadien-3,20-dion mit Lithiumdimethylcuprat umgelagert, aufgearbeitet und gereinigt. Ausbeute 3,9 g 11β,21-Dihydroxy-17-(3-methoxypropionyloxy)-1,4-pregnadien-3,20-dion.

c) Wie im Beispiel 5c beschrieben, wird 1,0 g 11β,21-Dihydroxy-17-(3-methoxypropionyloxy)-1,4-pregnadien-3,20-dion mit Essigsäureanhydrid bei Raumtemperatur umgesetzt, aufgearbeitet und gereinigt. Man isoliert 810 mg 21-Acetoxy-11β-hydroxy-17-(3-methoxypropionyloxy)-1,4-pregnadien-3,20-dion.

Beispiel 8

a) 15,0 g 11β,17,21-Trihydroxy-6α-methyl-1,4-pregnadien-3,20-dion werden analog Beispiel 1a mit Methoxyessigsäurechlorid umgesetzt, aufgearbeitet und gereinigt. Ausbeute 15,4 g 11β,17-Dihydroxy-21-methoxyacetoxy-6α-methyl-1,4-pregnadien-3,20-dion. Schmelzpunkt 219 °C.

b) Unter den Bedingungen des Beispiels 1b werden 15,0 g 11β,17-Dihydroxy-21-methoxyacetoxy-6α-methyl-1,4-pregnadien-3,20-dion mit Lithiumdimethylcuprat umgelagert und aufgearbeitet. Das Rohprodukt wird an 400 g Kieselgel mit einem Hexan-Aceton-Gradienten (0-50 % Aceton) gereinigt. Ausbeute 9,3 g 11β,21-Dihydroxy-17-methoxyacetoxy-6α-methyl-1,4-pregnadien-3,20-dion. Schmelzpunkt 202 °C.

Beispiel 9

2,0 g 11β,21-Dihydroxy-17-methoxyacetoxy-6α-methyl-1,4-pregnadien-3,20-dion werden unter den Bedingungen des Beispiels 5c mit Essigsäureanhydrid umgesetzt, aufgearbeitet und gereinigt. Ausbeute 1,6 g 21-Acetoxy-11β-hydroxy-17-methoxyacetoxy-6α-methyl-1,4-pregnadien-3,20-dion. Schmelzpunkt 151 °C.

Beispiel 10

a) Wie im Beispiel 2a beschrieben, werden 15,0 g 11β,17,21-Trihydroxy-6α-methyl-1,4-pregnadien-3,20-dion mit Äthoxyessigsäurechlorid umgesetzt, aufgearbeitet und gereinigt. Man isoliert 15,6 g 21-Äthoxyacetoxy-11β,17-dihydroxy-6α-methyl-1,4-pregnadien-3,20-dion. Schmelzpunkt 229 °C.

b) 15,0 g 21-Äthoxyacetoxy-11β,17-dihydroxy-6α-methyl-1,4-pregnadien-3,20-dion werden unter den Bedingungen des Beispiels 2b mit Lithiumdimethylcuprat umgelagert, aufgearbeitet und gereinigt. Ausbeute 8,3 g 17-Äthoxyacetoxy-11β,21-dihydroxy-6α-methyl-1,4-pregnadien-3,20-dion. Schmelzpunkt 198 °C.

Beispiel 11

2,0 g 17-Äthoxyacetoxy-11β,21-dihydroxy-6α-methyl-1,4-pregnadien-3,20-dion werden analog Beispiel 5c mit Essigsäureanhydrid umgesetzt, aufgearbeitet und gereinigt. Ausbeute 1,9 g 21-Acetoxy-17-äthoxyacetoxy-11β-hydroxy-6α-methyl-1,4-pregnadien-3,20-dion. Schmelzpunkt 170 °C.

Beispiel 12

a) Unter den Bedingungen des Beispiels 3a werden 15,0 g 11β,17,21-Trihydroxy-6α-methyl-1,4-pregnadien-3,20-dion mit 3-Methoxypropionsäurechlorid umgesetzt, aufgearbeitet und gereinigt. Man isoliert 14,3 g 11β,17-Dihydroxy-21-(3-methoxypropionyloxy)-6α-methyl-1,4-pregnadien-3,20-dion. Schmelzpunkt 230 °C.

b) 8,8 g 11β,17-Dihydroxy-21-(3-methoxypropionyloxy)-6α-methyl-1,4-pregnadien-3,20-dion werden analog Beispiel 3b mit Lithiumdimethylcuprat umgelagert, aufgearbeitet und gereinigt. Ausbeute 3,2 g 11β,21-Dihydroxy-17-(3-methoxypropionyloxy)-6α-methyl-1,4-pregnadien-3,20-dion. Schmelzpunkt 151 °C.

Beispiel 13

2,0 g 11β,21-Dihydroxy-17-(3-methoxypropionyloxy)-6α-methyl-1,4-pregnadien-3,20-dion werden analog Beispiel 5c mit Essigsäureanhydrid umgesetzt, aufgearbeitet und gereinigt. Man isoliert 1,36 g 21-Acetoxy-11β-hydroxy-17-(3-methoxypropionyloxy)-6α-methyl-1,4-pregnadien-3,20-dion. Schmelzpunkt 162 °C.

Beispiel 14

a) 15,0 g 11β,17,21-Trihydroxy-6α-methyl-1,4-pregnadien-3,20-dion werden analog Beispiel 4a mit 3-Äthoxypropionsäurechlorid umgesetzt, aufgearbeitet und gereinigt. Man isoliert 13,6 g 21-(3-Äthoxypropionyloxy)-11β,17-dihydroxy-6α-methyl-1,4-pregnadien-3,20-dion. Schmelzpunkt 186 °C.

b) Die Umlagerung von 10,0 g 21-(3-Äthoxypropionyloxy)-11β,17-dihydroxy-6α-methyl-1,4-pregnadien-3,20-dion mit Lithiumdimethylcuprat wird analog beispiel 4b durchgeführt. Nach der Aufarbeitung und Reinigung erhält man 4,1 g 17-(3-Äthoxypropionyloxy)-11β,21-dihydroxy-6α-methyl-1,4-pregnadien-3,20-dion. Schmelzpunkt 167 °C.

Beispiel 15

Wie im Beispiel 5c beschrieben, werden 2,0 g 17-(3-Äthoxypropionyloxy)-11β,21-dihydroxy-6α-methyl-1,4-pregnadien-3,20-dion mit Essigsäureanhydrid umgesetzt, aufgearbeitet und gereinigt. Ausbeute 1,34 g 21-Acetoxy-17-(3-äthoxypropionyloxy)-11β-hydroxy-6α-methyl-1,4-pregnadien-3,20-dion. Schmelzpunkt 131 °C.

Beispiel 16

1,0 g 11β,21-Dihydroxy-17-methoxyacetoxy-6α-methyl-1,4-pregnadien-3,20-dion werden analog Beispiel 5c mit Propionsäureanhydrid bei Raumtemperatur umgesetzt, aufgearbeitet und gereinigt. Man isoliert 780 mg 11β-Hydroxy-17-methoxyacetoxy-21-propionyloxy-6α-methyl-1,4-pregnadien-3,20-dion.

Beispiel 17

Unter den Bedingungen des Beispiels 5c werden 1,2 g 11β,21-Dihydroxy-17-methoxyacetoxy-6α-methyl-1,4-pregnadien-3,20-dion mit Buttersäureanhydrid bei Raumtemperatur umgesetzt, aufgearbeitet und gereinigt. Man erhält 730 mg 21-Butyryloxy-11β-hydroxy-17-methoxyacetoxy-6α-methyl-1,4-pregnadien-3,20-dion.

Beispiel 18

1,0 g 11β,21-Dihydroxy-17-methoxyacetoxy-6α-methyl-1,4-pregnadien-3,20-dion werden mit Isobuttersäureanhydrid unter den Bedingungen des Beispiels 5c umgesetzt, aufgearbeitet und gereinigt. Ausbeute 590 mg 11β-Hydroxy-21-isobutyryloxy-17-methoxyacetoxy-6α-methyl-1,4-pregnadien-3,20-dion.

Beispiel 19

3,0 g 11β,21-Dihydroxy-17-methoxyacetoxy-6α-methyl-1,4-pregnadien-3,20-dion werden in einem

Gemisch aus 225 ml Benzol und 75 ml Methanol mit 2,7 g Tris(triphenylphosphin)rhodium-I-chlorid 6,5 Stunden bei Raumtemperatur hydriert. Man engt im Vakuum zur Trockne ein und reinigt das Rohprodukt an 350 g Kieselgel mit einem Hexan-Aceton-Gradienten (0-50 % Aceton). Ausbeute 2,5 g 11β,21-Dihydroxy-17-methoxyacetoxy-6α-methyl-4-pregnen-3,20-dion.

Beispiel 20

Analog Beispiel 19 werden 1,5 g 21-Acetoxy-11β-hydroxy-17-methoxyacetoxy-6α-methyl-1,4-pregnadien-3,20-dion mit 1,2 g Tris(triphenylphosphin)rhodium-I-chlorid hydriert, aufgearbeitet und gereinigt. Ausbeute 1,1 g 21-Acetoxy-11β-hydroxy-17-methoxyacetoxy-6α-methyl-4-pregnen-3,20-dion.

Beispiel 21

Man hydriert, wie im Beispiel 19 beschrieben, 1,0 g 17-Äthoxyacetoxy-11β,21-dihydroxy-6α-methyl-1,4-pregnandien-3,20-dion mit 700 mg Tris(triphenylphosphin)rhodium-I-chlorid. Nach der Aufarbeitung und Reinigung isoliert man 790 mg 17-Äthoxyacetoxy-11β,21-dihydroxy-6α-methyl-4-pregnen-3,20-dion.

Beispiel 22

Unter den Bedingungen des Beispiels 19 werden 1,5 g 21-Acetoxy-11β-hydroxy-17-(3-methoxypropionyloxy)-6α-methyl-1,4-pregnadien-3,20-dion mit 1,2 g Tris(triphenylphosphin)rhodium-I-chlorid hydriert, aufgearbeitet und gereinigt. Ausbeute 1,2 g 21-Acetoxy-11β-hydroxy-17-(3-methoxypropionyloxy)-6α-methyl-4-pregnen-3,20-dion.

Beispiel 23

a) 1,5 g 11β,17,21-Trihydroxy-6α-methyl-4-pregnen-3,20-dion werden analog Beispiel 1a mit Methoxyessigsäurechlorid umgesetzt, aufgearbeitet und chromatographiert. Man isoliert 1,6 g 11β,17-Dihydroxy-21-methoxyacetoxy-6α-methyl-4-pregnen-3,20-dion.

b) 1,5 g 11β,17-Dihydroxy-21-methoxyacetoxy-6α-methyl-4-pregnen-3,20-dion werden unter den Bedingungen des Beispiels 1b mit Lithiumdimethylcuprat umgelagert, aufgearbeitet und gereinigt. Ausbeute 690 mg 11β,21-Dihydroxy-17-methoxyacetoxy-6α-methyl-4-pregnen-3,20-dion.

Beispiel 24

Wie in Beispiel 5c beschrieben, werden 600 mg 11β,21-Dihydroxy-17-methoxyacetoxy-6α-methyl-4-pregnen-3,20-dion mit Essigsäureanhydrid umgesetzt, aufgearbeitet und chromatographiert. Man isoliert 480 mg 21-Acetoxy-11β-hydroxy-17-methoxyacetoxy-6α-methyl-4-pregnen-3,20-dion.

**Patentansprüche**

1. Hydrocortison-Derivate der allgemeinen Formel I

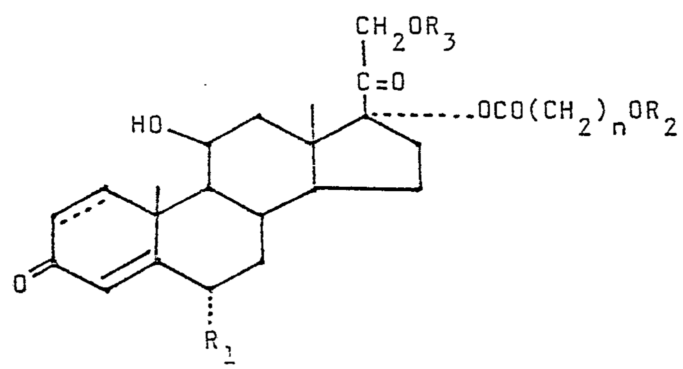

(I)

worin

..... eine Einfachbindung oder eine Doppelbindung,
n die Ziffern 1 oder 2,
$R_1$ ein Wasserstoffatom oder eine Methylgruppe,
$R_2$ einen Alkylrest mit 1 bis 6 Kohlenstoffatomen und
$R_3$ ein Wasserstoffatom oder einen 1-Oxoalkylrest mit 2 bis 6 Kohlenstoffatomen darstellen.

2. 17-Äthoxyacetoxy-11β,21-dihydroxy-4-pregnen-3,20-dion.

3. 11β,21-Dihydroxy-17-methoxyacetoxy-4-pregnen-3,20-dion.
4. 17-(3-Äthoxypropionyloxy)-11β,21-dihydroxy-4-pregnen-3,20-dion.
5. 11β,21-Dihydroxy-17-(3-methoxypropionyloxy)4-pregnen-3,20-dion.
6. 17-Äthoxyacetoxy-11β,21-dihydroxy-1,4-pregnandien-3,20-dion.
7. 21-Acetoxy-11β-hydroxy-17-(3-methoxypropionyloxy)-1,4-pregnadien-3,20-dion.
8. 11β,21-Dihydroxy-17-(3-methoxypropionyloxy)-6α-methyl-1,4-pregnadien-3,20-dion.
9. 21-Acetoxy-11β-hydroxy-17-(3-methoxypropionyloxy)-6α-methyl-1,4-pregnadien-3,20-dion.
10. 21-Acetoxy-11β-hydroxy-17-methoxyacetoxy-1,4-pregnadien-3,20-dion.
11. 17-Äthoxyacetoxy-11β,21-dihydroxy-6α-methyl-1,4-pregnadien-3,20-dion.
12. 17-(3-Äthoxypropionyloxy)-11β,21-dihydroxy-6α-methyl-1,4-pregnadien-3,20-dion.
13. 21-Acetoxy-17-(3-äthoxypropionyloxy)-11β-hydroxy-6α-methyl-1,4-pregnadien-3,20-dion.
14. 11β-Hydroxy-17-methoxyacetoxy-6α-methyl-21-propionyloxy-1,4-pregnadien-3,20-dion.
15. 21-Butyryloxy-11β-hydroxy-17-methoxyacetoxy-6α-methyl-1,4-pregnadien-3,20-dion.
16. 21-Acetoxy-11β-hydroxy-17-methoxymethoxy-6α-methyl-1,4-pregnadien-3,20-dion.
17. 11β-Hydroxy-21-isobutyryloxy-17-methoxyacetoxy-6α-methyl-1,4-pregnadien-3,20-dion.
18. 21-Acetoxy-17-äthoxyacetoxy-11β-hydroxy-6α-methyl-1,4-pregnadien-3,20-dion.
19. 11β,21-Dihydroxy-17-methoxyacetoxy-6α-methyl-4-pregnen-3,20-dion.
20. 17-Äthoxyacetoxy-11β,21-dihydroxy-6α-methyl-4-pregnen-3,20-dion.
21. 21-Acetoxy-11β-hydroxy-17-methoxyacetoxy-6α-methyl-4-pregnen-3,20-dion.
22. 21-Acetoxy-11β-hydroxy-17-(3-methoxypropionyloxy)-6α-methyl-4-pregnen-3,20-dion.
23. 11β,21-Dihydroxy-17-methoxyacetoxy-6α-methyl-1,4-pregnen-3,20-dion.
24. Pharmazeutische Präparate gekennzeichnet durch den Gehalt an ein oder zwei Hydrocortison-Derivaten gemäß Anspruch 1 bis 23.
25. Verfahren zur Herstellung von Hydrocortison-Derivaten der allgemeinen Formel I

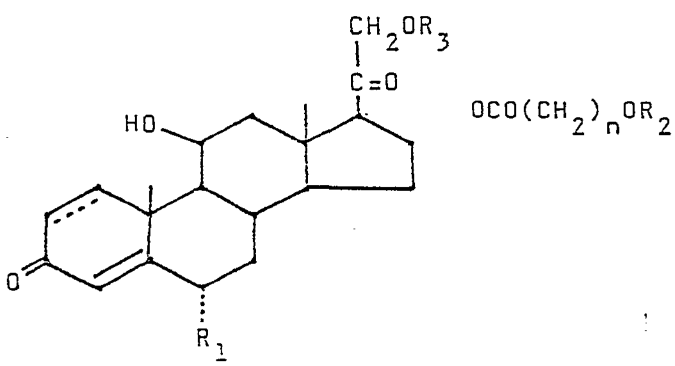

(I)

worin

..... eine Einfachbindung oder eine Doppelbindung,
n die Ziffern 1 oder 2,
$R_1$ ein Wasserstoffatom oder eine Methylgruppe,
$R_2$ einen Alkylrest mit 1 bis 6 Kohlenstoffatomen und
$R_3$ ein Wasserstoffatom oder einen 1-Oxoalkylrest mit 2 bis 6 Kohlenstoffatomen darstellen, dadurch gekennzeichnet, daß man ein Hydrocortison-21-acylat der allgemeinen Formel II

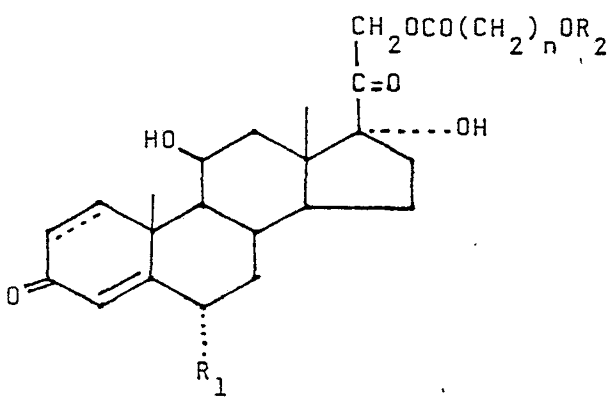

(II)

worin

....., $R_1$ und $R_2$ die obengenannte Bedeutung besitzen, zu dem entsprechenden 17-Acylat umlagert, gegebenenfalls in der 21-Position verestert und gewünschtenfalls die $\Delta^{1,4}$-Steroide der allgemeinen Formel I zu den $\Delta^4$-Steroiden hydriert.

**Claims**

1. Hydrocortisone derivatives of the general formula I

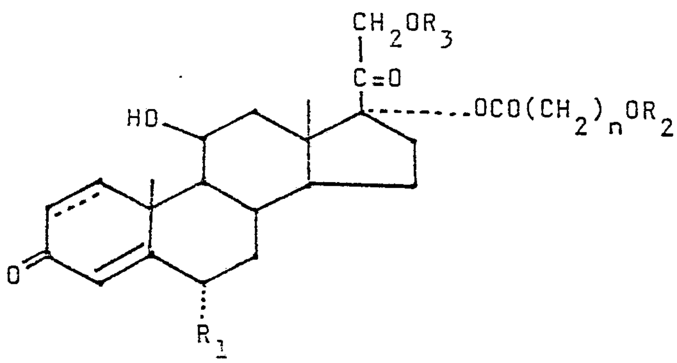

(I)

in which

..... is a single or double bond,
n is the integer 1 or 2,
$R_1$ is hydrogen or methyl,
$R_2$ is alkyl of 1 to 6 carbon atoms and
$R_3$ is hydrogen or 1-oxyalkyl of 2 to 6 carbon atoms.

2. 17-Ethoxyacetoxy-11β,21-dihydroxy-4-pregnene-3,20-dione.
3. 11β-21-Dihydroxy-17-methoxyacetoxy-4-pregnene-3,20-dione.
4. 17-(3-Ethoxypropionyloxy)-11β,21-dihydroxy-4-pregnene-3,20-dione.
5. 11β,21-Dihydroxy-17-(3-methoxypropionyloxy)-4-pregnene-3,20-dione.
6. 17-Ethoxyacetoxy-11β,21-dihydroxy-1,4-pregnadiene-3,20-dione.
7. 21-Acetoxy-11β-hydroxy-17-(3-methoxypropionyloxy)-1,4-pregnadiene-3,20-dione.
8. 11β,21-Dihydroxy-17-(3-methoxypropionyloxy)-α-methyl-1,4-pregnadiene-3,20-dione.
9. 21-Acetoxy-11β-hydroxy-17-(3-methoxypropionyloxy)-6α-methyl-1,4-pregnadiene-3,20-dione.
10. 21-Acetoxy-11β-hydroxy-17-methoxyacetoxy-1,4-pregnadiene-3,20-dione.
11. 17-Ethoxyacetoxy-11β,21-dihydroxy-6α-methyl-1,4-pregnadiene-3,20-dione.
12. 17-(3-Ethoxypropionyloxy)-11β,21-dihydroxy-6α-methyl-1,4-pregnadiene-3,20-dione.
13. 21-Acetoxy-17-(3-ethoxypropionyloxy)-11β-hydroxy-6α-methyl-1,4-pregnadiene-3,20-dione.
14. 11β-Hydroxy-17-methoxyacetoxy-6α-methyl-21-propionyloxy-1,4-pregnadiene-3,20-dione.
15. 21-Butyryloxy-11β-hydroxy-17-methoxyacetoxy-6α-methyl-1,4-pregnadiene-3,20-dione.
16. 21-Acetoxy-11β-hydroxy-17-methoxymethoxy-6α-methyl-1,4-pregnadiene-3,20-dione.
17. 11β-Hydroxy-21-isobutyryloxy-17-methoxyacetoxy-6α-methyl-1,4-pregnadiene-3,20-dione.
18. 21-Acetoxy-17-ethoxyacetoxy-11β-hydroxy-6α-methyl-1,4-pregnadiene-3,20-dione.
19. 11β,21-Dihydroxy-17-methoxyacetoxy-6α-methyl-4-pregnene-3,20-dione.
20. 17-Ethoxyacetoxy-11β,21-dihydroxy-6α-methyl-4-pregnene-3,20-dione.
21. 21-Acetoxy-11β-hydroxy-17-methoxyacetoxy-6α-methyl-4-pregnene-3,20-dione.
22. 21-Acetoxy-11β-hydroxy-17-(3-methoxypropionyloxy-6α-methyl-4-pregnene-3,20-dione.
23. 11β,21-Dihydroxy-17-methoxyacetoxy-6α-methyl-1,4-pregnene-3,20-dione.
24. Pharmaceutical compositions characterised in that they comprise one or two hydrocortisone derivatives according to claims 1 to 23.
25. Process for the preparation of hydrocortisone-derivatives of the general formula I

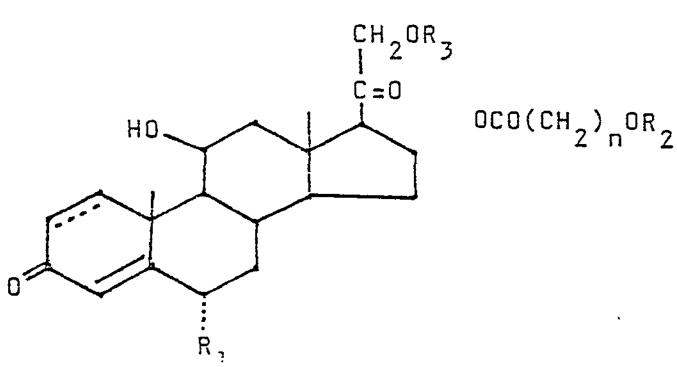

(I)

in which

..... is a single or double bond,

n is the integer 1 or 2,
$R_1$ is hydrogen or methyl,
$R_2$ is alkyl of 1 to 6 carbon atoms and
$R_3$ is hydrogen or 1-oxyalkyl of 2 to 6 carbon atoms characterised in that a hydrocortisone-21-acylate of general formula II

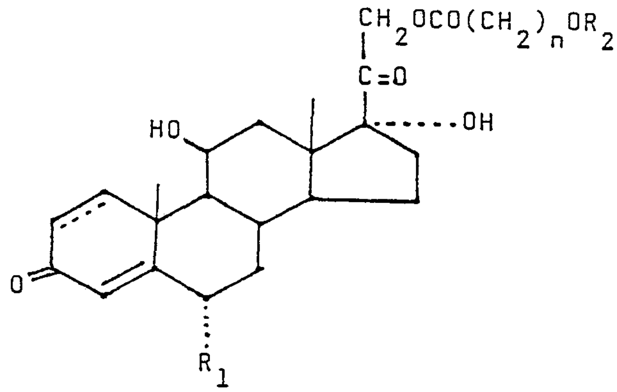

(II)

in which ....., $R_1$ and $R_2$ have the meanings given above, is rearranged to the corresponding 17-acylate and if necessary is esterified in the 21 position and optionally $\Delta^{1,4}$-steroids of general formula I are hydrogenated to $\Delta^4$-steroids.

**Revendications**

1. Dérivés de l'hydrocortisone répondant à la formule générale I :

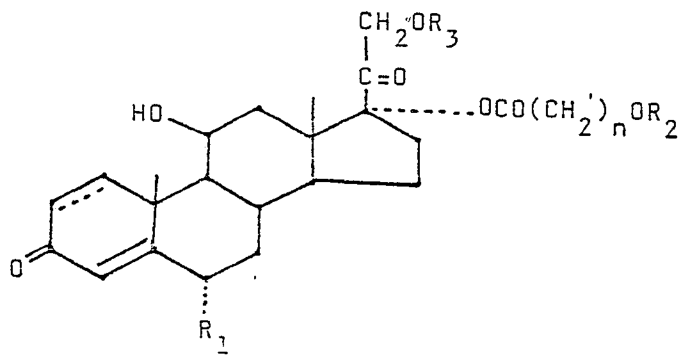

(I)

dans laquelle
..... représente une liaison simple ou une liaison double,
n représente le nombre 1 ou le nombre 2,
$R_1$ représente un atome d'hydrogène ou un radical méthyle,
$R_2$ représente un radical alkyle contenant de 1 à 6 atomes de carbone et
$R_3$ représente un atome d'hydrogène ou un radical alcanoyle contenant de 2 à 6 atomes de carbone.
2. Ethoxyacétoxy-17 dihydroxy-11β,21 prégnène-4 dione-3,20.
3. Dihydroxy-11β,21 méthoxyacétoxy-17 prégnène-4 dione-3,20.
4. (Ethoxy-3 propionyloxy)-17 dihydroxy-11β,21 prégnène-4 dione-3,20.
5. Dihydroxy-11β,21 (méthoxy-3 propionyloxy)-17 prégnène-4 dione-3,20.
6. Ethoxyacétoxy-17 dihydroxy-11β,21 prégnédiène-1,4 dione-3,20.
7. Acétoxy-21 hydroxy-11β (méthoxy-3 propionyloxy)-17 prégnadiène-1,4 dione-3,20.
8. Dihydroxy-11β,21 (méthoxy-3 propionyloxy)-17 méthyl-6α prégnadiène-1,4 dione-3,20.
9. Acétoxy-21 hydroxy-11β (méthoxy-3 propionyloxy)-17 méthyl-6α prégnadiène-1,4 dione-3,20.
10. Acétoxy-21 hydroxy-11β méthoxyacétoxy-17 prégnadiène-1,4 dione-3,20.
11. Ethoxyacétoxy-17 dihydroxy-11β,21 méthyl-6α prégnadiène-1,4 dione-3,20.
12. (Ethoxy-3 propionyloxy)-17 dihydroxy-11β,21 méthyl-6α prégnadiène-1,4 dione-3,20.
13. Acétoxy-21 (éthoxy-3 propionyloxy)-17 hydroxy-11β méthyl-6α prégnadiène-1,4 dione-3,20.
14. Hydroxy-11β méthoxyacétoxy-17 méthyl-6α propionyloxy-21 prégnadiène-1,4 dione-3,20.
15. Butyryloxy-21 hydroxy-11β méthoxyacétoxy-17 méthyl-6α prégnadiène-1,4 dione-3,20.
16. Acétoxy-21 hydroxy-11β méthoxyméthoxy-17 méthyl-6α prégnadiène-1,4 dione-3,20.

17. Hydroxy-11β isobutyryloxy-21 méthoxyacétoxy-17 méthyl-6α prégnadiène-1,4 dione-3,20.
18. Acétoxy-21 éthoxyacétoxy-17 hydroxy-11β méthyl-6α prégnadiène-1,4 dione-3,20.
19. Dihydroxy-11β,21 méthoxyacétoxy-17 méthyl-6α prégnène-4 dione-3,20.
20. Ethoxyacétoxy-17 dihydroxy-11β,21 méthyl-6α prégnène-4 dione-3,20.
21. Acétoxy-21 hydroxy-11β méthoxyacétoxy-17 méthyl-6α prégnène-4 dione-3,20.
22. Acétoxy-21 hydroxy-11β (méthoxy-3 propionyloxy)-17 méthyl-6α prégnène-4 dione-3,20.
23. Dihydroxy-11β,21 méthoxyacétoxy-17 méthyl-6α prégnadiène-1,4 dione-3,20.
24. Compositions pharmaceutiques caractérisées en ce qu'elles contiennent un ou deux dérivés de l'hydrocortisone selon l'une quelconque des revendications 1 à 23.
25. Procédé de préparations de dérivés de l'hydrocortisone répondant à la formule générale I :

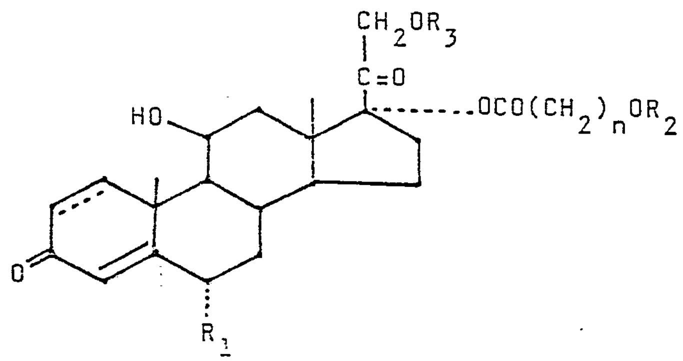

(I)

dans laquelle

..... représente une liaison simple ou une liaison double,

n représente le nombre 1 ou le nombre 2,

$R_1$ représente un atome d'hydrogène ou un radical méthyle,

$R_2$ représente un radical alkyle contenant de 1 à 6 atomes de carbone et

$R_3$ représente un atome d'hydrogène ou un radical alcanoyle contenant de 2 à 6 atomes de carbone, procédé caractérisé en ce qu'on transpose un acylate en 21 de l'hydrocortisone répondant à la formule générale II :

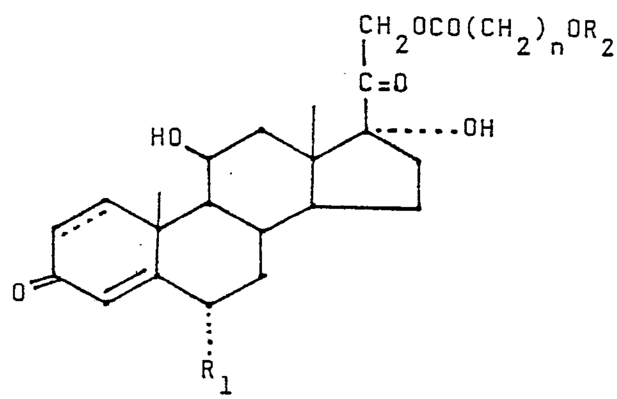

(II)

dans laquelle ....., $R_1$ et $R_2$ ont les significations précédemment données, de manière à le convertir en l'acylate en 17 correspondant, on estérifie éventuellement en position 21 et, si on le désire, on hydrogène les $\Delta^{1,4}$-stéroïdes de formule générale I de manière à les transformer en les $\Delta^4$-stéroïdes.